# EUROPÄISCHE PATENTSCHRIFT

(11) **EP 1 123 004 B1**
(45) Veröffentlichungstag und Bekanntmachung des Hinweises auf die Patenterteilung: **22.05.2002**
(21) Anmeldenummer: 99952494.5
(22) Anmeldetag: 05.10.1999
(51) Int. Cl.: A01N 63/02

(54) **VERWENDUNG EINES ANTIFUNGISCHEN PROTEINS AUS STREPTOMYCES TENDAE GEGEN PFLANZENPATHOGENE PILZE**
UTILIZATION OF AN ANTIFUNGAL PROTEIN FROM STREPTOMYCES TENDAE AGAINST PLANT PATHOGENIC FUNGI
UTILISATION D'UNE PROTEINE ANTIFONGIQUE DE STREPTOMYCES TENDAE POUR LUTTER CONTRE LES CHAMPIGNONS PATHOGENES DES PLANTES

(30) Priorität: 21.10.1998 DE 19848517
(43) Veröffentlichungstag der Anmeldung: 16.08.2001
(73) Patentinhaber: Aventis Research & Technologies GmbH & Co KG, 65926 Frankfurt am Main (DE)
(72) Erfinder: NEUMANN, Thomas, D-65929 Frankfurt (DE)
(74) Vertreter: Ackermann, Joachim, Dr.
(86) Internationale Anmeldenummer: EP9907364
(87) Internationale Veröffentlichungsnummer: WO0022932

(56) Entgegenhaltungen:
- DE-A- 2 537 028

## Beschreibung

Die vorliegende Erfindung betrifft die Verwendung eines antifungischen Proteins (AFP) mit einem Molekulargewicht von ca. 10 kDa aus Streptomyces tendae gegen pflanzenpathogene Pilze aus der Familie der Ascomyceten.

Seit längerer Zeit sind antifungische Proteine beschrieben worden, wobei einige dieser Proteine bei Pflanzen auch als PR-Proteine (Plant pathogenesis-related proteins) bezeichnet werden (Bol, J.F. & Linthorst, H.J.M. (1990), Annu. Rev. Phytopathol., 28, 113). Diese Proteine werden von Pflanzen unter Streßbedingungen, beispielsweise bei einer Virus- oder Pilzinfektion, gebildet, wodurch ein aktiver Verteidigungsmechanismus der Pflanze, der als induzierte Resistenz bezeichnet wird, induziert wird (Lindhorst, H.J.M. (1991) Cri. Rev. Plant Sci., 10 (2), 123). Einige dieser Proteine haben z.B. eine Chitinase- bzw. β-1,3-Glukanase-Aktivität.

Auch aus Mikroorganismen konnten bereits Proteine mit antifungischer Aktivität nachgewiesen werden, wobei einige dieser Proteine ebenfalls Chitinase- bzw. β-1,3 Glukanase-Aktivität zeigen. Andere Proteine wirken hingegen über eine Wechselwirkung mit der Pilzzellwand.

Aus Streptomyces tendae konnte beispielsweise ein antifungisches Protein (AFP) mit einer Größe von ca. 10 kDa isoliert werden, das gegen die Pilzarten Paecilomyces varriotii, Byssoclamis nivea, Penicillium puberulum und Eupenicillium terrum wirkt. Jedoch war die Wirkung auf diese Arten beschränkt. Andere Arten, wie z.B. Paecilomyces carneus, Paecilomyces lilacenus, Penicillium chrysogenum oder Penicillium claviforme werden nicht gehemmt. Darüber hinaus ist der Einsatz von AFP auch kommerziell nicht gerechtfertigt, da die genannten Pilzarten nicht pflanzenpathogen sind.

Aufgabe der vorliegenden Erfindung war es daher, ein antifungisches Protein zu finden, das gegen pflanzenpathogene Pilze wirksam ist.

Überraschenderweise wurde nun gefunden, daß AFP aus Streptomyces tendae gegen pflanzenpathogene Pilze der Familie der Ascomyceten wirksam ist.

Gegenstand der vorliegenden Erfindung ist daher die Verwendung eines antifungischen Proteins (AFP) mit einem Molekulargewicht von ca. 10 kDa aus Streptomyces tendae gegen pflanzenpathogene Pilze aus der Familie der Ascomyceten, insbesondere gegen Botrytis cinera.

Das genannte antifungische Protein enthält eine typische aminoterminale Signalsequenz, d.h. auf einen hydrophilen N-Terminus folgt eine hydrophobe Transmembranregion, die eine extrazelluläre Sekretion bewirken. Zudem sind zwei Formen bekannt, eine kürzere Form mit einem Molekulargewicht von ca. 9.860 Da und eine längere Form mit einem Molekulargewicht von ca. 10.300 Da.

Da AFP von Streptomyces tendae in das Kulturmedium sekretiert wird, kann dieses beispielsweise direkt aus dem Kulturfiltrat von Streptomyces tendae nach dem Fachmann bekannten Methoden isoliert werden. Alternativ kann AFP gentechnisch hergestellt werden, wobei das isolierte Gen vorzugsweise in einen sogenannten Multicopyvektor, z.B. plJ702 (Hopwood, D.A. et al. (1985) Genetic Manipulation of Streptomyces. A Laboratory Manual. Norwich, U.K.) kloniert wird und damit ein geeigneter Stamm, beispielsweise der Nikkomycin-Nichtproduzentenstamm Streptomyces tendae NP9 transformiert wird. Ein Beispiel für eine AFP-kodierende Nukleinsäure ist in SEQ ID No. 1 wiedergegeben. Wurde diese bevorzugte Transformate beispielsweise in 200 ml Nährlösung für 7 Tage angezogen, so konnten ca. 6 mg AFP, entsprechend 30 mg AFP/I, gewonnen werden. Die gentechnische Herstellung von AFP über eine vorzugsweise kontrollierte Fermentation ist daher besonders bevorzugt.

Für die erfindungsgemäße Verwendung kann AFP beispielsweise in Form einer Formulierung mit vorzugsweise mindestens einem zusätzlichen Hilfsmittel, direkt eingesetzt werden. Hierzu werden beispielsweise pilzbefallene oder durch Pilzbefall gefährdete Pflanzen mit der beschriebenen Formulierung besprüht. Die Konzentration von AFP in der Formulierung ist im allgemeinen ca. 10 ug/ml bis zu ca. 500 mg/ml. Als Hilfsmittel eignen sich beispielsweise Proteaseinhibitoren, Stabilisatoren wie Glycerin, Saccharose, Salze, Lösungsmittel oder allgemein bekannte Substanzen aus dem Pflanzenschutz.

Eine andere Schutzmöglichkeit vor Pilzbefall bieten sogenannte transgene Pflanzen, die das AFP selbst herstellen können.

Eine weitere Ausführungsform ist daher die erfindungsgemäße Verwendung von AFP, wobei das AFP in einer transgenen Pflanze gebildet wird. Die transgene Pflanze ist vorzugsweise gentechnisch veränderter Mais, Baumwolle, Kartoffel, Banane, Arabidopsis, Casava, Tabak, Raps (Canola), Kartoffel, Zuckerrübe, Getreide, wie z.B. Weizen, Gerste oder Hafer, Erbeeren, Gemüse, wie z.B. Kohl, Hülsenfrüchte, wie z.B. Erbsen oder Bohnen, Tomate, Salat oder Melone.

Zur Herstellung einer transgenen Pflanze, wird eine Nukleinsäure kodierend für AFP beispielsweise in Form von nackter DNA, viraler DNA oder RNA, oder in Form von Plasmid-DNA in eine Pflanzenzelle eingebracht. Ein Beispiel für eine AFP-kodierende Nukleinsäure ist in SEQ ID No. 1 wiedergegeben. Gemäß der vorliegenden Erfindung werden jedoch auch solche Nukleinsäuren erfaßt, die aufgrund der Entartung des genetischen Codes von der Nukleinsäuresequenz gemäß SEQ ID No. 1 verschieden sind, jedoch für die selbe AFP-Aminosäuresequenz kodieren. Des weiteren werden solche Mutanten oder Varianten der Nukleinsäure gemäß SEQ ID No. 1 erfaßt, die für ein AFP-Protein kodieren, welches gegen pflanzenpathogene Pilze der Familie der Ascomyceten, insbesondere gegen Botrytis cinera, wirksam ist. Hierunter fallen beispielsweise Fusionsproteine des AFP-Proteins mit anderen Fremdproteinen oder ein AFP-Protein mit einem N-terminal deletierten Methionin. Weitere Beispiele von Varianten sind Nukleinsäuren, die unter stringenten Bedingungen mit der Nukleinsäure gemäß SEQ ID No. 1 hybridisieren. Die stringenten Hybridisierungsbedingungen lassen sich beispielsweise nach Sambrook, J. et al. in Molecular Cloning, A Laboratory Manual, 2^{nd} Edition, Cold Spring Habour Laboratory Press, 1989 bestimmen. Anschließend werden aus den transformierten Pflanzenzellen transgene Pflanzen regeneriert. Vorzugsweise kann eine AFP-kodierende Nukleinsäure mittels rekombinanten Agrobakterien, Elektroporation, Beschuß mit Mikroprojektilen und/oder Polyethylenglykol in die Pflanzenzelle eingebracht werden.

Die Infektion mit rekombinanten Agrobacterium tumefaciens-Bakterien in Pflanzenzellen von Dikotyledonen ist beispielsweise bei Klee, H. et.al. (1997) Annu. Rev. Plant Physiol. 38, 467 oder EP-B2-0122791) beschrieben. Die Infektion mit rekombinanten Agrobacterium tumefaciens-Bakterien in Pflanzenzellen von Monokotyledonen ist beispielsweise bei Ishida, Y. et.al. (1996) Nature Biotech. 14, 745 am Beispiel vom Mais (Zea mays L.) beschrieben. Die zu diesem Zweck verwendeten Agrobakterien besitzen eine T-DNA, in welche das AFP-exprimierende Gen und gegebenenfalls ein geeigneter Promotor, beispielsweise ein Pflanzen-Phaseolinpromotor (siehe z.B. EP-B2-0122791) oder ein viraler 35S-Promotor (siehe z.B. Ishida, Y. et.al. (1996), supra) inseriert wurde. Die Übertragung der T-DNA in Pflanzenzellen kann beispielsweise dadurch geschehen, daß man die rekombinanten Agrobakterien zusammen mit unreifen Embryonen der betreffenden Pflanze cokultiviert (siehe z.B. Ishida, Y. et.al. (1996) supra). Zur Selektion der transformierten Pflanzenzellen werden vorzugsweise T-DNA-Konstrukte verwendet, die in der Lage sind, aus dem zu exprimierenden AFP-Gen ein Resistenzgen in der Pflanzenzelle zu exprimieren. Als Resistenzgen eignet sich beispielsweise das Gen kodierend für die Phosphinothricin-Acetyltransferase (siehe z.B. Ishida, Y. et.al. (1996), supra). Erfolgreich transformierte Pflanzenzellen können somit beispielsweise durch das entsprechende Antibiotikum Phosphinothricin selektiert werden. Die Regeneration einer Pflanze aus transformierten Pflanzenzellen ist allgemein bekannt (siehe z.B. Sagi et.al. (1995), Nature Biotech, 13, 481-485, Ishida, Y. et.al. (1996), supra oder Schöpke et.al. (1996), Nature Biotech, 14, 731-735.

Neben der Verwendung von Agrobacterium tumefaciens als Transformationsmittel sind weitere Transformationsverfahren bekannt und geeignet, wie z.B. die Elektroporation, der Beschuß mit Mikroprojektilen oder die Verwendung von Polyethylenglycol (siehe z.B. Potrykos, (1991) Annu. Rev. Plant Physiol. Mol. Biol., 42, 205; Estruch, J.J. et.al. (1997), Nature Biotech., 15, 137-141 oder Dingermann, T. (1995) BlOforum, 18, 252). Beispielsweise konnte durch Beschuß von Pflanzenzellen mit DNA-beschichteten Mikroprojektilen bereits Casava (Schöpke et.al. (1996), supra) oder Banane (Sagi, et.al. (1995), supra) transformiert werden. Auch bei diesem Verfahren wird das zu exprimierende AFP-Gen in einen geeigneten Vektor kloniert, der vorzugsweise ein Resistenzgen besitzt, welches die spätere Selektion der transformierten Pflanzenzellen ermöglicht. Der rekombinante Vektor wird vorzugsweise auf Wolframprojektile aufgebracht, die beispielsweise in embryogenische Zellsuspensionen geschossen werden (siehe z.B. Sagi, et.al, (1995), supra). Anschließend werden die so behandelten Zellsuspensionen mit einem entsprechenden Antibiotikum, beispielsweise Phosphinothricin, auf transformierte Pflanzenzellen selektioniert und hieraus eine transgene Pflanze, wie beispielsweise oben bereits beschrieben, regeneriert.

Ein wesentlicher Vorteil der vorliegenden Erfindung ist, daß AFP gegen pflanzenpathogene Pilze aus der Familie der Ascomyceten und insbesondere gegen Botrytis Cinera wirkt, die vor allem bereits gegen ein oder mehrere Pflanzenschutzmittel resistent geworden sind.

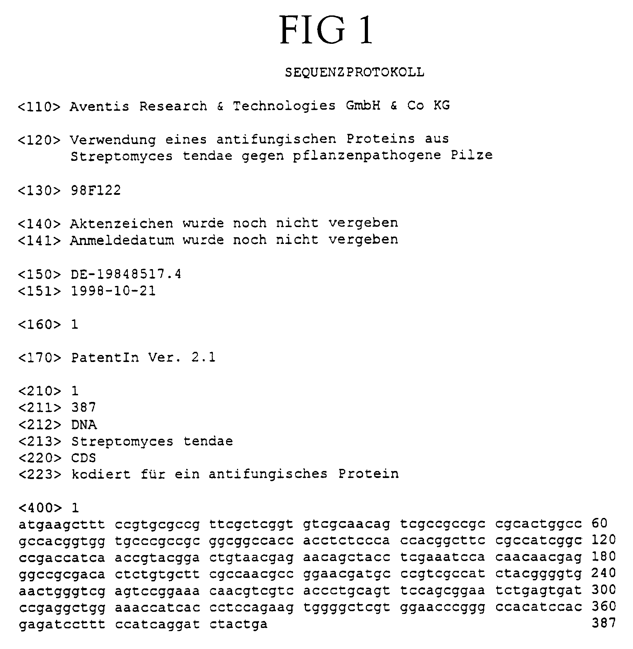

Die nachfolgenden Beispiele und die Figur sollen die Erfindung näher beschreiben, ohne sie zu beschränken:

### Beschreibung der Sequenz

- SEQ ID No. 1: zeigt eine Nukleinsäuresequenz kodierend für ein antifungisches (223) Protein (AFP) aus Streptomyces tendae.

### Beispiele

### Beispiel 1:

Zur Herstellung von Testplatten wurden 50 µl einer Sporensuspension von *Botrytis cinerae* zu 100 ml Test-Agar (20 g/l Malzextrakt, 10 g/l Glukose, 2 g/l Hefeextrakt, 0,5 Ammoniumsulfat, 15 g/l Agar, pH 6.0) gegeben. Davon wurden 25 ml in Petrischalten gegossen. Die Endkonzentration der Sporen betrug 10⁵ Sporen / ml. Anschließend wurden sterile Antibiotikatestplättchen (Schleicher und Schuell, Dassel, Frg) mit einem Durchmesser von 0,5 cm auf einer Testplatte gelegt. Danach pipettierte man 5, 10, 15 und 20 µl der AFP-Lösung (100 mg / ml lyophilisiertes Kulturfiltrat von S. tendae-Transformanten) auf die Testplättchen und inkubierte die Testplatte für 4 Tage bei 30°C. Die Hemmhofdurchmesser betrug für 5 ul AFP-Lösung 0,7 cm, für 10 µl 1 cm Durchmesser, für 15 µl Lösung 1,3 cm und für 20 µl 1,9 cm.

### Beispiel 2:

Zur Herstellung von Testplatten wurden 50 µl einer Sporensuspension von *Botrytis cinerae* ZF 3629 (resistent gegenüber BCM (Benlat, Benomyl™, Hoechst Schering AgrEvo GmbH, Frankfurt, Carbendazem, ) zu 100 ml Test-Agar (20 g/l Malzextrakt, 10 g/I Glukose, 2 g/l Hefeextrakt, 0,5 Ammoniumsulfat, 15 g/l Agar, pH 6.0) gegeben. Davon wurden 25 mi in Petrischalten gegossen. Die Endkonzentration der Sporen betrug 10⁵ Sporen / ml. Anschließend wurden sterile Antibiotikatestplättchen (Schleicher und Schuell, Dassel, Frg) mit einem Durchmesser von 0,5 cm auf eine Testplatte gelegt. Danach pipettierte man 5, 10, 15 und 20 µl der AFP-Lösung (100 mg / ml lyophilisiertes Kulturfiltrat von S. *tendae*-Transformanten) auf die Testplättchen und inkubierte die Testplatte für 4 Tage bei 30°C. Die Hemmhofdurchmesser betrug für 5 µl AFP-Lösung 0,9 cm, für 10 µl 1,3 cm, für 15 µl Lösung 1,9 cm und für 20 µl 2,2 cm.

### Beispiel 3 - Verbesserte Herstellung durch Fermentation:

100 ml des Vorkulturmediums (103 g/l Saccharose, 20 g Tryptic Soy Broth (Oxoid, Wesel), 10 g/l MgCl₂ und 10 g/l Hefeextrakt) in einem Schikanekolben wurden mit einem Agarstück des Stammes *S*. *tendae* beimpft. Die Kultur erfolgte für 5 Tage bei 28°C und 200 upm auf einer rotierenden Schüttelmaschine.
10 I des Produktionsmediums (103 g/l Saccharose, 20 g Tryptic Soy Broth (Oxoid, Wesel), 10 g/l MgCl₂, 10 g/l Hefeextrakt, 100 µl Desmophen, pH 7,5) wurden mit 100 ml der oben beschriebenen Vorkultur beimpft.
Als Fermenter wurde ein Biostat E (Braun, Melsungen) eingesetzt. Die Fermentation erfolgte bei 28°C, 700 upm und 0,5 vvm Luft über 4 Tage. Danach wurde die Fermentation gestoppt und durch Zentrifugation bei 3000 upm und 4°C der Kulturüberstand geerntet. Anschließend wurde der Ansatz durch Lyophilisation getrocknet. Das Pulver konnte direkt eingesetzt werden. Wurden 15 µl des Lyophilisats (100 mg/ml) in einem Hemmhoftest gegen *Botrytis cinerae* eingesetzt, konnte ein Hemmhof von 1,7 cm und gegen *Botrytis cinerae ZF* 3629 ein Hemmhof von 2,3 cm nachgewiesen werden.

## Patentansprüche

1. Verwendung eines antifungischen Proteins (AFP) mit einem Molekulargewicht von ca. 10 kDa aus Streptomyces tendae gegen pflanzenpathogene Pilze aus der Familie der Ascomyceten, insbesondere gegen Botrytis cinera.

2. Verwendung nach Anspruch 1, **dadurch gekennzeichnet, daß** AFP in Form einer Formulierung mit vorzugsweise mindestens einem Hilfsmittel verwendet wird.

3. Verwendung nach Anspruch 1, **dadurch gekennzeichnet, daß** AFP in einer transgenen Pflanze gebildet wird.

4. Verwendung nach Anspruch 3, **dadurch gekennzeichnet, daß** die transgene Pflanze ausgewählt ist aus Mais, Baumwolle, Kartoffel, Banane, Arabidopsis, Casava, Tabak, Raps (Canola), Kartoffel, Zuckerrübe, Getreide, Erbeeren, Gemüse, Hülsenfrüchte, Tomate, Salat oder Melone.

5. Verwendung nach Anspruch 4, **dadurch gekennzeichnet, daß** Getreide ausgewählt ist aus Weizen, Gerste oder Hafer, als Gemüse Kohl verwendet wird oder die Hülsenfrüchte ausgewählt sind aus Erbsen oder Bohnen.

6. Verwendung nach einem der Ansprüche 3 - 5, **dadurch gekennzeichnet, daß** die transgene Pflanze eine Nukleinsäure gemäß SEQ ID No. 1 enthält, wobei SEQ ID No. 1 Teil des Anspruchs ist.

7. Verwendung nach einem der Ansprüche 1 - 6, **dadurch gekennzeichnet, daß** der genannte pflanzenpathogene Pilz gegen ein oder mehrere Pflanzenschutzmittel resistent ist.

## Claims

1. The use of an antifungal protein (AFP) having a molecular weight of approx. 10 kDA from Streptomyces tendae against plant pathogenic fungi from the Ascomycetes family, in particular against Botrytis cinera.

2. The use as claimed in claim 1, wherein AFP is used in the form of a formulation together with preferably at least one auxiliary.

3. The use as claimed in claim 1, wherein AFP is formed in a transgenic plant.

4. The use as claimed in claim 3, wherein the transgenic plant is selected from among maize, cotton, potato, banana, Arabidopsis, casava, tobacco, oilseed rape (canola), potato, sugar beet, cereals, strawberries, vegetables, legumes, tomato, lettuce or melon.

5. The use as claimed in claim 4, wherein cereals are selected from amongst wheat, barley or oats, the vegetable used is cabbage, or the legumes are selected from amongst peas or beans.

6. The use as claimed in any of claims 3 - 5, wherein the transgenic plant comprises a nucleic acid as claimed in SEQ ID No. 1, SEQ ID No. 1 being part of the claim.

7. The use as claimed in any of claims 1 - 6, wherein the plant pathogenic fungus stated is resistant to one or more crop protection agents.

## Revendications

1. Utilisation d'une protéine antifongique (PAF) d'un poids moléculaire d'environ 10 kDa à partir de *Streptomyces tendae* contre des champignons phytopathogènes de la famille des *Ascomycetes,* en particulier contre *Botrytis cinerae.*

2. Utilisation selon la revendication 1, **caractérisée en ce qu'**on utilise la PAF sous forme d'une formulation de préférence conjointement avec au moins un adjuvant.

3. Utilisation selon la revendication 1, **caractérisée en ce que** la PAF est formée dans une plante transgénique.

4. Utilisation selon la revendication 3, **caractérisée en ce que** la plante transgénique est pris dans le groupe comportant le maïs, le coton, la pomme de terre, la banane, l'arabidopsis, la cassave, le tabac, le colza (canola), la pomme de terre, la betterave, des céréales comme, les fraises, des légumes, des légumes secs, la tomate, la salade ou le melon.

5. Utilisation selon la revendication 4, **caractérisée en ce que** les céréales sont pris parmi le blé, l'orge ou l'avoine, en tant que légume, on utilise le choux ou les légumes secs sont choisis parmi les petits poids ou les haricots.

6. Utilisation selon l'une des revendications 3 à 5, **caractérisée en ce que** la plante transgénique renferme un acide nucléique selon la SEQ ID NO 1, la SEQ ID NO 1 faisant partie de la revendication.

7. Utilisation selon l'une des revendications 1 à 6, **caractérisée en ce que** le champignon phytopathogène cité est résistant contre un ou plusieurs agents phytoprotecteurs.
